(19) Europäisches Patentamt / European Patent Office / Office européen des brevets

(11) **EP 4 043 456 A1**

(12) **EUROPEAN PATENT APPLICATION**

(43) Date of publication:
**17.08.2022 Bulletin 2022/33**

(21) Application number: **22150924.3**

(22) Date of filing: **11.01.2022**

(51) International Patent Classification (IPC):
***C07D 413/12*** *(2006.01)* ***A01N 43/80*** *(2006.01)*

(52) Cooperative Patent Classification (CPC):
(C-Sets available)
**C07D 413/12; A01N 43/80; A01P 13/00** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.02.2021 GB 202101909**

(71) Applicant: **Rotam Agrochem International Company Limited**
**Hong Kong (HK)**

(72) Inventor: **BRISTOW, James Timothy**
**N/A Chai Wan (HK)**

(74) Representative: **Akers, Noel James**
**N.J. Akers & Co**
**63 Lemon Street**
**Truro, Cornwall TR1 2PN (GB)**

# (54) CRYSTALLINE FORM OF PYROXASULFONE, METHODS FOR ITS PREPARATION AND USE OF THE SAME

(57) A novel crystalline modification of Pyroxasulfone is provided, characterized by an X-ray powder diffractogram (XRD), an infrared (IR) spectrum, a melting point and/or a differential scanning calorimetry (DSC) profile. There is also provided a method for preparing the crystalline modification of Pyroxasulfone comprising: i) providing a solution of Pyroxasulfone in a solvent system comprising a one or more solvents; ii) precipitating the crystalline modification I of Pyroxasulfone from the solution; and iii) isolating the precipitated crystalline modification I of Pyroxasulfone. Compositions comprising the crystalline modification I of Pyroxasulfone and the use of the crystalline modification I in the control of unwanted plant growth are also provided.

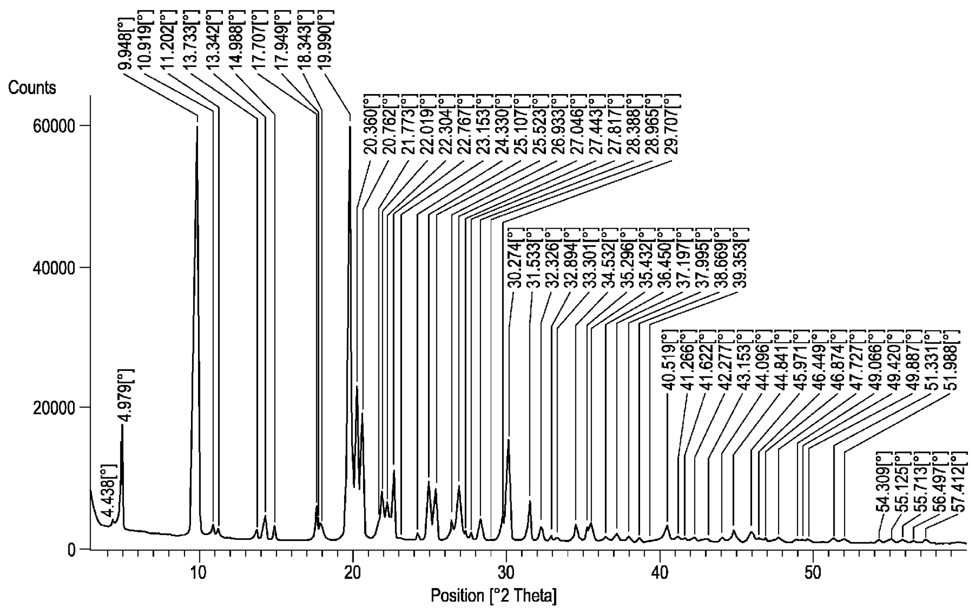


Figure 1

EP 4 043 456 A1

(52) Cooperative Patent Classification (CPC): (Cont.)

C-Sets
**A01N 43/80, A01N 25/12**

## Description

The present invention relates to a novel crystalline form of 3-[[[5-(difluoromethoxy)-1-methyl-3-(trifluoromethyl)-1H-pyrazol-4-yl]methyl]sulfonyl]-4,5-dihydro-5,5-dimethylisoxazole (Pyroxasulfone). Further, the present invention relates to methods for the preparation of the novel crystalline form of Pyroxasulfone. Still further, the present invention relates to the use of the novel crystalline form of Pyroxasulfone in agrochemical preparations and for the control of unwanted plant growth.

WO 2021/002484 discloses a group of sulfone derivatives, methods for their preparation and their use as herbicides.

3-[[[5-(difluoromethoxy)-1-methyl-3-(trifluoromethyl)-1H-pyrazol-4-yl]methyl]sulfonyl]-4,5-dihydro-5,5-dimethylisoxazole, having the common name Pyroxasulfone, is a pyrazole herbicide Pyroxasulfone is highly effective against a wide range of grass and broadleaf weeds in a wide range of crops, including maize, soybeans, wheat and triticale. Pyroxasulfone has the molecular formula $C_{12}H_{14}F_5N_3O_2S$ and its chemical structure can be represented as follows:

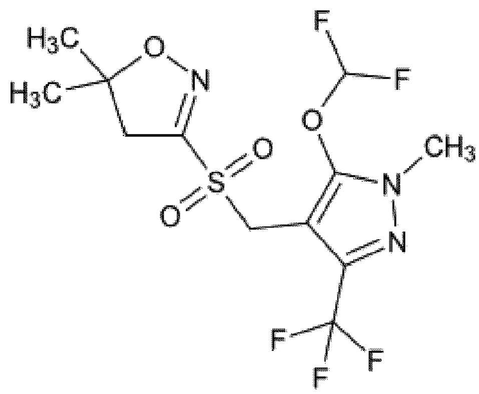

Pyroxasulfone and formulations containing Pyroxasulfone are available commercially. Pyroxasulfone is usually manufactured by the process described in US 7,256,298 and it is present in an amorphous state where the melting point is 129-130°C. However, it has been found that the commercially available Pyroxasulfone exhibits a significant lack of dispersibility and storage stability, reducing the effectiveness and suitability of the compound as an active component in herbicide compositions and formulations. As a result, the herbicidal activity of the compound decreases. This in turn requires larger amounts of Pyroxasulfone to be applied in shorter intervals in order to achieve a required level of herbicidal activity, leading to the production of potentially toxic degradation products.

CN111393427A concerns a method for preparing Pyroxasulfone. The method comprises synthesizing a range of intermediates in an extensive reaction scheme. In the final step, Pyroxasulfone is prepared by the reaction of an intermediate in a solvent in the presence of a catalyst system and hydrogen peroxide. The solvent is selected from dichloromethane, methanol and dichloroethane.

WO 2020/240392 discloses a process scheme for the preparation of Pyroxasulfone and a range of intermediates. The process is said to provide the intermediates and Pyroxasulfone in high yields.

Therefore, there is a need to provide a novel form of Pyroxasulfone having improved properties. It would be advantageous if a form of Pyroxasulfone could be provided having increased dispersibility, as well as improved storage stability.

It is known that some organic compounds exist in only one crystalline form, while other compounds can exist in two or more crystalline forms. It is not possible to predict the number of different crystalline forms a given compound will have, nor the physical, chemical and biological properties of the different crystalline forms, which may be markedly different.

It has now been discovered that Pyroxasulfone can exist in a crystalline form, herein termed "crystalline modification I", which has been found to exhibit significantly improved properties. In particular the crystalline modification I of Pyroxasulfone exhibits a high resistance to hydrolysis. This provides significant advantages when using Pyroxasulfone in the crystalline modification I when preparing agrochemical formulations and the use thereof in the control of unwanted plant growth.

Accordingly, in a first aspect, the present invention provides a crystalline modification I of Pyroxasulfone, the crystalline modification I exhibiting at least three of the following reflexes, in any combination, as $2\theta \pm 0.2$ degree in an X-ray powder diffractogram (XRD) recorded using Cu-K$\alpha$ radiation at 25 °C:

$$2\theta = 9.94 \pm 0.2 \quad (1)$$

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.02 \pm 0.2 \quad (5)$$

$$2\theta = 22.77 \pm 0.2 \quad (6)$$

$$2\theta = 25.11 \pm 0.2 \quad (7)$$

$$2\theta = 25.52 \pm 0.2 \quad (8)$$

$$2\theta = 27.05 \pm 0.2 \quad (9)$$

$$2\theta = 30.27 \pm 0.2 \quad (10)$$

$$2\theta = 31.53 \pm 0.2 \quad (11)$$

In a second aspect, the present invention provides a crystalline modification I of Pyroxasulfone, the crystalline modification I exhibiting an infrared (IR) spectrum with characteristic functional group vibration peaks at wavenumbers ($cm^{-1}$, $\pm$ 0.2%) of one or more of 2987.05, 1572.09, 1488.51, 1375.02, 1329.55, 1251.06, 1168.65, 1125.87, 1102.30 and 1050.31 $cm^{-1}$.

In a third aspect, the present invention provides a crystalline modification I of Pyroxasulfone, the crystalline modification I exhibiting a melting point of from 131.7 to 134.1°C.

In a fourth aspect, the present invention provides a crystalline modification I of Pyroxasulfone, the crystalline modification I exhibiting a differential scanning calorimetry (DSC) profile having an endothermic melting peak with onset at 131.7°C and peak maximum at 134.1°C.

The crystalline modification I of Pyroxasulfone is useful in controlling plant growth. Accordingly, the present invention also provides compositions for controlling plant growth, such as weeds, comprising the crystalline modification I of Pyroxasulfone. In the compositions, Pyroxasulfone may be employed on its own, as a mixture with auxiliaries and carriers and/or as a mixture with other active compounds.

In a further aspect, the present invention provides the use of the crystalline modification I of Pyroxasulfone in the control of undesirable plant growth.

Still further, the present invention provides a method for controlling plant growth at a locus, the method comprising applying to the locus the crystalline modification I of Pyroxasulfone.

The aspects of the present invention relating to the crystalline modification I of Pyroxasulfone will be described in more detail hereinbelow, having reference where appropriate, to the accompanying figures, in which:

Figure 1 is an X-ray powder diffraction (XRD) spectrum of the crystalline modification I of Pyroxasulfone of the present invention;

Figure 2 is an infrared (IR) spectrum of the crystalline modification I of Pyroxasulfone of the present invention;

Figure 3 is a Differential Scanning Calorimetry (DSC) spectrum of the crystalline modification I of Pyroxasulfone of the present invention; and

Figure 4 is an X-ray powder diffraction (XRD) spectrum of the amorphous Pyroxasulfone of the prior art.

As noted above, it has been found that the crystalline modification I of Pyroxasulfone exhibits a significant improvement in its dispersibility and storage stability, which significantly increase the effectiveness and suitability of Pyroxasulfone, when compared with the form of Pyroxasulfone as used in the current commercially available formulations. In addition, it has been found that the crystalline modification I of Pyroxasulfone is easier to comminute and/or grind than known forms of Pyroxasulfone. This facilitates the preparation of a wide range of agrochemical formulations, such as suspension concentrates (SC), oil-based suspension concentrates (OD), water-dispersible granules (WG) and water-soluble granules (SG).

As noted above, in one embodiment, the crystalline modification I of Pyroxasulfone may be characterized as exhibiting at least three of the following reflexes, in any combination, as $2\theta \pm 0.2$ degree in an X-ray powder diffractogram (XRD) recorded using Cu-K$\alpha$ radiation at 25 °C:

$$2\theta = 9.94 \pm 0.2 \quad (1)$$

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.02 \pm 0.2 \quad (5)$$

$$2\theta = 22.77 \pm 0.2 \quad (6)$$

$$2\theta = 25.11 \pm 0.2 \quad (7)$$

$$2\theta = 25.52 \pm 0.2 \quad (8)$$

$$2\theta = 27.05 \pm 0.2 \quad (9)$$

$$2\theta = 30.27 \pm 0.2 \quad (10)$$

$$2\theta = 31.53 \pm 0.2 \quad (11)$$

The crystalline modification I of Pyroxasulfone of the present invention is characterized by an X-ray powder diffractogram having at least three of the reflexes indicated above. Preferably, the crystalline modification I of Pyroxasulfone is one having at least four of the aforementioned reflexes, more preferably at least five of the aforementioned reflexes, still more preferably six, more preferably still at least seven, especially at least eight of the aforementioned reflexes, again in any combination thereof.

In one embodiment, the crystalline modification I of Pyroxasulfone exhbits all of the reflexes indicated above. An X-ray powder diffractogram of the crystalline modification I of Pyroxasulfone is shown in Figure 1, which will be described in detail hereinafter.

In one preferred embodiment, the crystalline modification I of Pyroxasulfone exhibits at least three, more preferably four, still more preferably five, more preferably still six, especially seven, of the following reflexes, in any combination, as 2θ±0.2 degree in an X-ray powder diffractogram (XRD) recorded using Cu-Kα radiation at 25°C:

$$2\theta = 9.94 \pm 0.2 \quad (1)$$

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.02 \pm 0.2 \quad (5)$$

$$2\theta = 22.77 \pm 0.2 \quad (6)$$

$$2\theta = 25.11 \pm 0.2 \quad (7)$$

$$2\theta = 27.05 \pm 0.2 \quad (9)$$

$$2\theta = 30.27 \pm 0.2 \quad (10)$$

In a preferred embodiment, the crystalline modification I of Pyroxasulfone exhibits at least one, preferably at least two, more preferably at least three, still more preferably at least four of the following reflexes, in any combination, as 2θ±0.2 degree in an X-ray powder diffractogram recorded using Cu-Kα radiation at 25°C:

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.77 \pm 0.2 \quad (6)$$

$$2\theta = 30.27 \pm 0.2 \quad (10)$$

A preferred crystalline modification I of Pyroxasulfone exhibits all of the reflexes (2) to (10) listed above.

The X-ray powder diffractogram of the crystalline modification I of Pyroxasulfone shown in Figure 1 was taken using a diffractometer with a reflection geometry in the range from 3° to 60° with increments of 0.03° using Cu-Ka radiation at 25°C.

As noted above, in a second aspect, the present invention provides a crystalline modification I of Pyroxasulfone exhibiting an infrared (IR) spectrum with characteristic functional group vibration peaks at wavenumbers ($cm^{-1}$, ± 0.2%) of one or more of the following: 2987.05, 1572.09, 1488.51, 1375.02, 1329.55, 1251.06, 1168.65, 1125.87, 1102.30 and 1050.31 $cm^{-1}$.

An infrared (IR) spectrum of the crystalline modification I of Pyroxasulfone is shown in Figure 2.

All IR spectra data were obtained using the following acquisition parameters:

| | |
|---|---|
| FT-IR spectrometer | Bruker Tensor37 |
| Diamond ATR unit | from Specac |
| Wavelength range | 550 - 4000 $cm^{-1}$ |
| Resolution | 4 $cm^{-1}$ |
| Number of scans | 16 |

A preferred crystalline modification I of Pyroxasulfone exhibits an infrared (IR) spectrum with characteristic functional group vibration peaks at wavenumbers ($cm^{-1}$, ± 0.2%) of two or more, preferably three or more, more preferably four or more, still more preferably five or more of the following: 2987.05, 1572.09, 1488.51, 1375.02, 1329.55, 1251.06, 1168.65, 1125.87, 1102.30 and 1050.31 $cm^{-1}$. A preferred crystalline modification II of Pyroxasulfone exhibits an infrared (IR) spectrum with characteristic functional group vibration peaks at wavenumbers ($cm^{-1}$, ± 0.2%) having six or more, preferably seven or more, more preferably eight or more, still more preferably nine or more, especially all of the aforementioned vibration peaks.

In one embodiment, the crystalline modification I of Pyroxasulfone exhibits an X-ray powder diffractogram as described above for the first aspect of the invention and an infrared (IR) spectrum as described above for the second aspect of the present invention.

As discussed above, a third aspect of the present invention provides a crystalline modification I of Pyroxasulfone, exhibiting a melting point of from 131.7 to 134.1°C, preferably a melting point of about 133.3°C.

In one embodiment, the crystalline modification I of Pyroxasulfone exhibits a melting point of from 131.7 to 134.1°C according to this third aspect of the invention, together with an X-ray powder diffractogram as described above for the first aspect of the invention and/or an infrared (IR) spectrum as described above for the second aspect of the present invention.

As also discussed above, a fourth aspect of the present invention provides a crystalline modification I of Pyroxasulfone exhibiting a differential scanning calorimetry (DSC) profile having an endothermic melting peak with onset at 131.7°C and peak maximum at 133.3°C, more preferably with a melting enthalpy of 39.95 J/g.

In one embodiment, the crystalline modification I of Pyroxasulfone exhibits a differential scanning calorimetry (DSC) profile having an endothermic melting peak with onset at 131.7°C and peak maximum at 133.3°C according to this fourth aspect of the invention, together with an X-ray powder diffractogram as described above for the first aspect of the invention and/or an infrared (IR) spectrum as described above for the second aspect of the present invention and/or a melting point as described above for the third aspect of the present invention.

In one preferred embodiment, the crystalline modification I of Pyroxasulfone is characterized by an X-ray powder diffraction pattern substantially as shown in Figure 1, and/or characterized by an IR spectrum substantially as shown in Figure 2, and/or characterized by a DSC thermogram substantially as shown in Figure 3, and/or by a melting point of about 133.3°C.

Pyroxasulfone is available commercially. Methods for preparing the known forms of Pyroxasulfone are well known in the art. One particularly suitable method for preparing the known forms of Pyroxasulfone is described in US 7,256,298.

In a fifth aspect, the present invention provides a method for preparing a crystalline modification I of Pyroxasulfone, the method comprising the steps of:

i) providing a solution of Pyroxasulfone in a solvent system comprising one or more solvents;

ii) precipitating the crystalline modification I of Pyroxasulfone from the solution; and

iii) isolating the precipitated crystalline modification I of Pyroxasulfone.

The solution of Pyroxasulfone may be provided in step i) by dissolving Pyroxasulfone in the solvent system. The form of Pyroxasulfone used in this step may be any form of Pyroxasulfone other than the crystalline modification I. In one embodiment Pyroxasulfone dissolved in the solvent system in step i) is amorphous Pyroxasulfone.

The solvent system employed in the method is one in which Pyroxasulfone is readily soluble and is one from which the crystalline modification I of Pyroxasulfone is crystallised. In this respect, it has been found that appropriate selection of the solvent system is required in order to yield Pyroxasulfone in the crystalline modification I. The solvent system yielding the crystalline modification I of Pyroxasulfone comprises one or more solvents selected from ethers, for

example, ethyl propyl ether, n-butyl ether, anisole, phenetole, cyclohexyl methyl ether, dimethyl ether, dimethyl glycol, diphenyl ether, dipropyl ether, diisopropyl ether, di-n-butyl ether, diisobutyl ether, diisoamyl ether, ethylene glycol dimethyl ether, isopropyl ethyl ether, methyl tert-butyl ether, tetrahydrofuran, methyl-tetrahydrofuran, dichlorodiethyl ether, methyl-tetrahydrofuran, polyethers of ethylene oxide and/or propylene oxide; and esters, for example malonates, n-butyl ester (n-butyl acetate), methyl acetate, isobutyl acetate, dimethyl carbonate, diethyl carbonate, dibutyl carbonate and ethylene carbonate.

Preferably the solvent system comprises one or more solvents selected from dichlorodiethyl ether, methyl tert-butyl ether, methyl-tetrahydrofuran, malonates, n-butyl acetate, isobutyl acetate, diethyl carbonate. Particularly preferred solvents are dichlorodiethyl ether and n-butyl acetate.

As noted above, it has been found that the crystalline form of Pyroxasulfone obtained by crystallization from a solution in a solvent system comprising one or more of the aforementioned solvents exhibits a significant and surprisingly increased in dispersibility and storage stability.

Accordingly, in a further aspect, the present invention provides the use of a solvent system to prepare crystalline Pyroxasulfone having an improved dispersibility and storage stability, wherein the solvent system comprises one or more solvents selected from ethers and esters.

The solvent system may consist essentially of a single solvent selected from the aforementioned solvents. Alternatively, the solvent system may comprise a mixture of two or more of the aforementioned solvents, for example a mixture of three or four solvents.

In step i) of the method, a solution of Pyroxasulfone in the solvent system is provided. As noted above, this may be achieved by dissolving Pyroxasulfone in the solvent system. To form the solution, Pyroxasulfone may be dissolved in the solvent system at any suitable temperature. It is preferred to heat the solvent system to a temperature from ambient temperature to the reflux temperature of the solvent system. In one embodiment, Pyroxasulfone is dissolved in the solvent system at the reflux temperature of the solvent system. In one embodiment, the solvent system is heated to a temperature from 20 to 70°C, more preferably from 30 to 60°C. The temperature will depend upon such factors as the solubility of Pyroxasulfone in the solvent system and the boiling point of the solvent system.

In step ii) of the method, Pyroxasulfone is caused to precipitate from the solution to yield the crystalline modification I of Pyroxasulfone. Any suitable technique for precipitating Pyroxasulfone from the solution provided in step i) may be used. In one embodiment, the solution is cooled. The solution may be cooled to any suitable temperature to cause the crystalline modification I of Pyroxasulrone to precipitate out of the solution, such as a temperature below room or ambient temperature to promote precipitation of crystalline Pyroxasulfone. For example, the solution may be cooled to a temperature of from -20 to 10°C, preferably to a temperature of from -15 to 5°C.

Alternatively, or in addition to cooling, precipitation may be facilitated by removing solvent from the solution, for example by applying a vacuum to the solution.

In one embodiment seed crystals are added to the solution. The addition of seed crystals facilitates precipitation of the solute from the solution, as is known in the art. Preferably, the seed crystals are crystals of Pyroxasulfone, more preferably crystals of the crystalline modification I of Pyroxasulfone.

The amount of seed crystals added to the solution is typically in the range of from 0.001 to 10% by weight, preferably from 0.001% to 2.5% by weight, more preferably from 0.005 to 0.5% by weight, based on the weight of Pyroxasulfone present in the solution provided in step i). The seed crystals are preferably added to the solution at a temperature below the boiling point of the solvent system.

In step iii) of the method, the precipitated crystalline modification I of Pyroxasulfone is isolated or recovered from the solvent system. Any suitable technique may be used to recover the crystalline modification I of Pyroxasulfone, for example filtration, centrifugation and/or decantation.

The isolated solid Pyroxasulfone is preferably washed one or more times with a solvent system comprising one or more solvents. Preferably, the solvent system employed in the washing stage comprises one or more components of the solvent system of the solution provided in step (i), as described hereinbefore. Washing is preferably carried out using the solvent system at a temperature from 0°C to room temperature, depending on the solubility of the crystalline form of Pyroxasulfone in the solvent, in order to minimize or avoid the loss of crystalline material in the corresponding washing solvent.

The use of Pyroxasulfone as a herbicide is known in the art and is used on a commercial scale. It has been found that the crystalline modification I of Pyroxasulfone is also active in controlling undesirable plant growth, such as weeds. Techniques of formulating and applying amorphous Pyroxasulfone are known in the art, for example as disclosed in the prior art documents described hereinbefore. These techniques can also be applied in an analogous manner to the crystalline modification I of Pyroxasulfone.

Accordingly, the present invention further provides a herbicidal composition comprising the crystalline modification I of Pyroxasulfone as defined hereinbefore.

The herbicidal composition generally comprises one or more auxiliary components, as described in more detail hereinafter.

The herbicidal composition may comprise the crystalline modification I of Pyroxasulfone in any suitable amount, which may depend upon such factors as the type of formulation being employed. Preferably, the composition comprises the crystalline modification I of Pyroxasulfone in an amount of up to 90% by weight of the composition, preferably up to 80% by weight of the composition, more preferably up to 40% by weight of the composition, more preferably up to 10% by weight of the composition. Preferably, the composition comprises the crystalline modification I of Pyroxasulfone in an amount of from 10% by weight of the composition, preferably from 20% by weight of the composition, more preferably from 30% by weight of the composition. In one preferred embodiment, the composition comprises the crystalline modification I of Pyroxasulfone in an amount of about 40% by weight of the composition.

The composition may be formulated in any suitable form. Preferably, the composition is in the form of a suspension concentrate (SC), an oil-based suspension concentrate (OD), water-soluble granules (SG), a dispersible concentrate (DC), an emulsifiable concentrate (EC), an emulsion seed dressing, a suspension seed dressing, granules (GR), microgranules (MG), a suspoemulsion (SE) or water-dispersible granules (WG). The components and techniques required to form the aforementioned formulations are known in the art.

In one preferred embodiment, the composition is in the form of water-dispersible granules (WG).

In one preferred embodiment, the composition is in the form of a suspension concentrate (SC).

The compositions are prepared by combining the crystalline modification I of Pyroxasulfone with one or more agriculturally acceptable auxiliaries. The auxiliaries employed in the composition and their amounts will depend upon the type of formulation and/or the manner in which the formulation is to be applied by the end user. Suitable auxiliaries are customary formulation adjuvant or components, such as dispersants, wetting agents, emulsifiers, extenders, carriers, solvents, surfactants, stabilizers, anti-foam agents, anti-freeze agents, preservatives, antioxidants, colourants, thickeners, solid adherents and inert fillers. Such auxiliaries are known in the art and are commercially available. Their use in the formulation of the compositions of the present invention will be apparent to the person skilled in the art.

Surfactants can be an emulsifier, dispersant or wetting agent of ionic or nonionic type. Examples which may be used include, but are not limited to, salts of polyacrylic acids, salts of lignosulphonic acid, salts of phenylsulphonic or naphthalenesulphonic acids, polycondensates of ethylene oxide with fatty alcohols or with fatty acids or with fatty amines, substituted phenols, especially alkylphenols, sulphosuccinic ester salts, taurine derivatives, especially alkyltaurates, or phosphoric esters of polyethoxylated phenols or alcohols.

Liquid diluents include, but are not limited to, water, N,N-dimethylamide, dimethyl sulfoxide, N-alkylpyrrolidone, ethylene glycol, polypropylene glycol, propylene carbonate, dibasic esters, paraffins, alkylbenzenes, alkyl naphthalenes, glycerine, triacetine, oils of olive, castor, linseed, sesame, corn, peanut, cotton-seed, soybean, rape-seed and coconut, ketones such as cyclohexanone, 2-heptanone, isophorone and 4-hydroxy-4-methyl-2-pentanone, acetates such as hexyl acetate, heptyl acetate and octyl acetate, and alcohols such cyclohexanol, decanol, benzyl and tetrahydrofurfuryl alcohol, and mixtures thereof.

The composition may further comprise one or more polymeric stabilizers. Suitable polymeric stabilizers that may be used in the present invention include, but are not limited to polypropylene, polyisobutylene, polyisoprene, copolymers of monoolefins and diolefins, polyacrylates, polystyrene, polyvinyl acetate, polyurethanes or polyamides. Suitable stabilizers are known in the art and are commercially available.

The composition may further comprise one or more anti-foam agents. Suitable anti-foam agents include those substances which can normally be used for this purpose in agrochemical compositions and will be readily apparent to the person skilled in the art. Suitable anti-foam agents are known in the art and are commercially available. Particularly preferred anti-foam agents are mixtures of polydimethylsiloxanes and perfluroalkylphosphonic acids, such as the silicone anti-foam agents (for example commercially available from GE or Compton). Other examples of anti-foam agents are fatty acids, tallow, and sodium salts.

The composition may further comprise one or more preservatives. Suitable preservatives include those substances which can normally be used for this purpose in agrochemical compositions of this type and again are well known in the art. Suitable examples that may be mentioned include Preventol® (commercially available from Bayer AG) and Proxel® (commercially available from Bayer AG).

The composition may further comprise one or more antioxidants. Suitable antioxidants are substances which can normally be used for this purpose in agrochemical compositions, as is known in the art. Preference is given, for example, to butylated hydroxytoluene.

The composition may further comprise one or more solid adherents. Such adherents are known in the art and available commercially. Suitable solid adherents include organic adhesives, including tackifiers, such as celluloses of substituted celluloses, natural and synthetic polymers in the form of powders, granules, or lattices, and inorganic adhesives such as gypsum, silica, or cement.

The composition may further comprise one or more inert fillers. Such inert fillers are known in the art and available commercially. Suitable fillers include, for example, natural ground minerals, such as kaolins, aluminas, talc, chalk, quartz, attapulgite, montmorillonite, and diatomaceous earth, or synthetic ground minerals, such as highly dispersed silicic acid, aluminum oxide, silicates, and calcium phosphates and calcium hydrogen phosphates. Suitable inert

fillers for granules include, for example, crushed and fractionated natural minerals, such as calcite, marble, pumice, sepiolite, and dolomite, or synthetic granules of inorganic and organic ground materials, as well as granules of organic material, such as sawdust, coconut husks, corn cobs, and tobacco stalks. Examples of inert fillers also include sodium tripolyphosphate and sucrose.

Solid diluents can be water-soluble or water-insoluble. Water-soluble solid diluents include, but are not limited to, salts such as alkali metal phosphates (for example sodium dihydrogen phosphate), alkaline earth phosphates, sulfates of sodium, potassium, magnesium and zinc, sodium and potassium chloride, sodium acetate, sodium carbonate and sodium benzoate, and sugars and sugar derivatives such as sorbitol, lactose, sucrose and mannitol. Examples of water-insoluble solid diluents include, but are not limited to clays, synthetic and diatomaceous silicas, calcium and magnesium silicates, titanium dioxide, aluminum, calcium and zinc oxide, and mixtures thereof.

Wetting agents include, but are not limited to, alkyl sulfosuccinates, laureates, alkyl sulfates, phosphate esters, acetylenic diols, ethoxyfluornated alcohols, ethoxylated silicones, alkyl phenol ethyoxylates, benzene sulfonates, alkyl-substituted benzene sulfonates, alkyl a-olefin sulfonates, naphthalene sulfonates, alkyl-substituted naphthalene sulfonates, condensates of naphthalene sulfonates and alkyl-substituted naphthalene sulfonates with formaldehyde, and alcohol ethoxylates, and mixtures thereof. Alkyl naphthalene sulphonates, sodium salts are particularly useful for the composition of the invention.

Dispersants include, but are not limited to, sodium, calcium and ammonium salts of ligninsulfonates (optionally polyethoxylated); sodium and ammonium salts of maleic anhydride copolymers; sodium salts of condensed phenolsulfonic acid; and naphthalene sulfonate-formaldehyde condensates. Ligninsulfonates such as sodium ligninsulfonates are particularly useful for the composition of the invention. Naphthalene sulfonate-formaldehyde condensates such as naphthalenesulfonic acid, polymers with formaldehyde, and sodium salts are particularly useful for the composition of the invention.

Thickening agents include, but are not limited to, guar gum, pectin, casein, carrageenan, xanthan gum, alginates, methylcellulose, hydroxyethylcellulose, hydroxypropylcellulose, and carboxymethylcellulose, and mixtures thereof. Synthetic thickening agents include derivatives of the former categories, and also polyvinyl alcohols, polyacrylamides, polyvinylpyrrolidones, various polyethers, their copolymers, as well as polyacrylic acids and their salts, and mixtures thereof. Alkylpolyvinylpyrrolidones are particularly useful for the composition of the invention.

Other formulation components can also be used in the present invention such as dyes, drying agents, and the like. These components and their uses are known to one skilled in the art.

The composition of the present invention may comprise the crystalline modification I of Pyroxasulfone as the sole active ingredient. Alternatively, other active components may be present, such as attractants, sterilizing agents, bactericides, acaricides, nematicides, fungicides, growth-regulating substances, herbicides, safeners, fertilizers, semiochemicals, insecticides or agents for improving plant properties.

Preferred mixing partners of the crystalline modification I of Pyroxasulfone are carfentrazone-ethyl, chlorimuron-ethyl+flumioxazin, flumioxazin, fluthiacet-methyl, fluthiacet-methyl + atrazine, imazethapyr+saflufenacil.

The present invention also provides a method for controlling unwanted plant growth, comprising applying to the plant, plant part, or surroundings of the plant, a herbicidally effective amount of the crystalline modification I of Pyroxasulfone as hereinbefore described.

The crystalline modification I of Pyroxasulfone is preferably applied in the form of a composition as hereinbefore described.

Methods and techniques for applying the compositions of the present invention are known in the art and will be understood by the person skilled in the art. Techniques include diluting or dispersing the composition in a suitable diluent or carrier liquid, in particular water, and applying the composition by spraying.

All plants, plant parts, and their surroundings can be treated with the crystalline modification I of Pyroxasulfone in accordance with the present invention. In the present context, plants are to be understood as meaning all plants and plant populations such as desired and undesired wild plants or crop plants, including naturally occurring crop plants. Crop plants can be plants which can be obtained by conventional breeding and optimization methods, by biotechnological and genetic engineering methods, or by combinations of these methods, including the transgenic plants and the plant cultivars which may or may not be protected by plant breeders' rights.

Plant parts are to be understood as meaning all parts and organs of plants above and below the ground, such as shoots, leaves, needles, stalks, stems, flowers, fruit bodies, fruits, seeds, roots, tubers and rhizomes. Harvested materials, and vegetative and generative propagation materials, for example, cuttings, tubers, meristem tissue, rhizomes, offsets, seeds, single and multiple plant cells and any other plant tissues, are also included.

Treatment of the plants, plant parts, and/or their surroundings, with the compositions or formulations of the invention can be carried out directly or by allowing the compositions or formulations to act on their surroundings, habitat or storage space by the customary treatment methods known in the art. Examples of these customary treatment methods include dipping, spraying, vaporizing, fogging, broadcasting, painting on in the case of propagation material, and applying one or more coats particularly in the case of seeds.

The benefits of the present invention are particularly advantageous when the crystalline modification I of Pyroxasulfone or its herbicidal composition are applied to kill weeds in crops of useful plants, such as maize, soybeans, wheat, triticale, corn, cotton, beans, peanuts, potatoes, rape, garlic, tobacco, sunflower, castor-oil plants and scallion,. The crop plants are preferably maize, soybeans, wheat and triticale.

The invention may be used to control a wide range of undesired plants, including broadleaf plants and grassy weeds, in particular annual gramineous weeds such as crab grass, green bristlegrass, goosegrass herb, barnyard grass, moleplant seed, alopecurus, wild oat, blue grass, stiff grass, teff and the like, and broadleaf weeds such as chenopodiaceae, amaranthaceae, polygonaceae, daygrass, hamamelis, dodder and the like.

Throughout the description and claims of this specification, the words "comprise" and variations of the words, for example "comprising" and "comprises", mean "including but not limited to", and do not exclude other moieties, additives, components, integers or steps. Moreover the singular encompasses the plural unless the context otherwise requires: in particular, where the indefinite article is used, the specification is to be understood as contemplating plurality as well as singularity, unless the context requires otherwise.

In this specification, references to properties are - unless stated otherwise - to properties measured under ambient conditions, i.e. at atmospheric pressure and at a temperature of about 20°C.

As used herein, the term "about" or "around" when used in connection with a numerical amount or range, means somewhat more or somewhat less than the stated numerical amount or range, and for example to a deviation of ± 10% of the stated numerical amount or endpoint of the range.

"Surrounding," as used herein, refers to the place on which the plants are growing, the place on which the plant propagation materials of the plants are sown or the place on which the plant propagation materials of the plants will be sown.

"Precipitation" as used herein, refers to the sedimentation of a solid material (a precipitate), including the sedimentation of a crystalline material, from a liquid solution in which the solid material is present in amounts greater than its solubility in the amount of liquid solution.

The term "herbicidally effective amount" as used herein, refers to the quantity of such a compound or combination of such compounds that is capable of producing a controlling effect on the growth of plants. The controlling effects include all deviation from the natural development of the target plants, for example killing, retardation of one or more aspects of the development and growth of the plant, leaf burn, albinism, dwarfing and the like.

Embodiments of the present invention will now be described by way of the following examples, which are provided for illustrative purposes only.

All percentages are given in weight percent, unless otherwise indicated.

## EXAMPLES

### Example 1: Preparation of commercial Pyroxasulfone in accordance with the disclosure of US 7,256,298 (Reference Examples 1 and 3)

Production of 3-(5-difluoromethoxy-1-methyl-3-trifluoromethyl-1H-pyrazole-4-ylmethylthio)-5,5-dimethyl-2-isoxazoline

To a solution of 6.7 g (35.0 mmol) of 3-ethanesulfonyl-5,5-dimethyl-2-isoxazoline in 50 ml of N,N-dimethylformamide was added 5.6 g (purity: 70%, 70.0 mmol) of sodium hydrosulfide, followed by 1 hour of stirring at room temperature. Thereafter, 4.8 g (35.0 mmol) of potassium carbonate and 10.8 g (35.0 mmol) of 4-bromomethyl-5-difluoromethoxy-1-methyl-3-trifluoromethyl-1H-pyrazole were added thereto, followed by stirring at room temperature overnight. After the completion of the reaction was confirmed, the reaction solution was poured into water and extracted with ethyl acetate. The resulting organic layer was washed with water and saline and then dried over anhydrous magnesium sulfate. The solvent was removed by evaporation under reduced pressure and the residue was purified by silica gel column chromatography to obtain 7.3 g (yield: 57.9%) of 3-(5-difluoromethoxy-1-methyl-3-trifluoromethyl-1H-pyrazole-4-ylmethylthio)-5,5-dimethyl-2-isoxazoline as white crystals (melting point: 39 to 40° C).

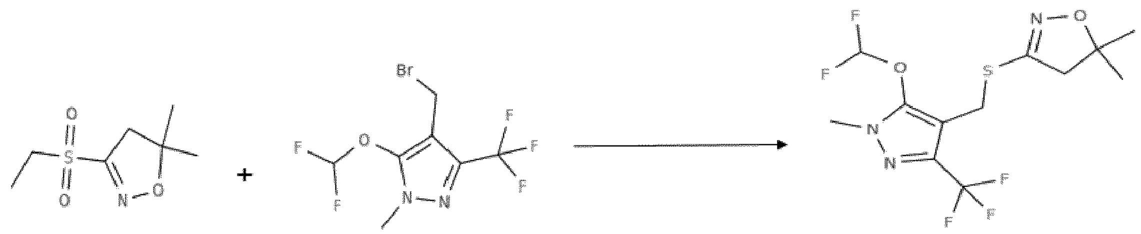

3-ethanesulfonyl-5,5-dimethyl-2-isoxazoline

4-bromomethyl-5-difluoromethoxy-1-methyl-3-trifluoromethyl-1H-pyrazole

3-(5-difluoromethoxy-1-methyl-3-trifluoromethyl-1H-pyrazole-4-ylmethylthio)-5,5-dimethyl-2-isoxazoline

Production of 3-(5-difluoromethoxy-1-methyl-3-trifluoromethyl-1H-pyrazole-4-yl-methanesulfonyl)-5,5-dimethyl-2-isoxazoline (Pyroxasulfone)

To a solution of 7.3 g (20.3 mmol) of 3-(5-difluoromethoxy-1-methyl-3-trifluoromethyl-1H-pyrazole-4-ylmethylthio)-5,5-dimethyl-2-isoxazoline in 50 ml of chloroform was added 12.5 g (purity: 70%, 50.8 mmol) of m-chloroperbenzoic acid under ice-cooling, followed by 1 hour of stirring. Thereafter, the whole was further stirred at room temperature overnight. After the completion of the reaction was confirmed, the reaction solution was poured into water and extracted with chloroform. The resulting organic layer was washed with an aqueous sodium hydrogen sulfite solution, an aqueous sodium hydrogen carbonate, water, and saline, successively, and then dried over anhydrous magnesium sulfate. The solvent was removed by evaporation under reduced pressure and the resulting solid was washed with n-hexane to obtain 6.4 g (yield: 80.6%) of 3-(5-difluoromethoxy-1-methyl-3-trifluoromethyl-1H-pyrazole-4-yl-methanesulfonyl)-5,5-dimethyl-2-isoxazoline (Pyroxasulfone) as a white powder (melting point: 129 to 130° C).

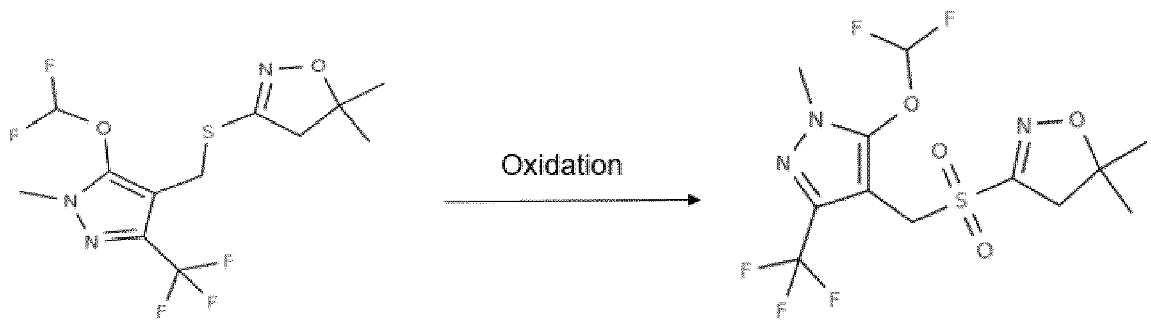


3-(5-difluoromethoxy-1-methyl-3-trifluoromethyl-1H-pyrazole-4-ylmethylthio)-5,5-dimethyl-2-isoxazoline

Pyroxasulfone

The solid Pyroxasulfone product was isolated by filtering with suction. The Pyroxasulfone obtained was confirmed to be amorphous.

**Example 2**

**Preparation of crystalline modification I of Pyroxasulfone (crystallization from dichlorodiethyl ether)**

Pyroxasulfone prepared in Example 1 (10 g) was placed in a 3 neck round bottom flask, together with dichlorodiethyl ether (60 mL) and stirred. The resulting slurry was heated to 90°C to achieve a homogeneous solution. The insoluble particles, if any, were removed by filtration. The remaining solution was slowly cooled to room temperature. Upon cooling, fine crystals formed. The slurry of crystals and solution was stirred at room temperature for 2 hours. Thereafter, the slurry was filtered and washed with dichlorodiethyl ether (3 mL). The filtered crystals were dried under vacuum at room temperature in order to remove the dichlorodiethyl ether traces from the crystalline product.

The crystalline product thus obtained had a purity of >98% and the product recovered as crystals was found to be not less than 80% yield.

The crystal product was analyzed by IR spectrometry, XRD and DSC and found to be the crystalline modification

I of Pyroxasulfone as shown in Figures 1, 2 and 3, respectively.

The X-ray diffractogram of the crystal exhibited the reflexes as shown in Figure 1 and the values are summarized in Table 1 below.

Table 1

| Crystalline modification I of Pyroxasulfone | |
|---|---|
| 2θ(°) | d(Å) |
| 2θ = 9.94 ± 0.2 | 8.89 ± 0.05 |
| 2θ = 19.95 ± 0.2 | 4.45 ± 0.05 |
| 2θ = 20.36 ± 0.2 | 4.36 ± 0.05 |
| 2θ = 20.76 ± 0.2 | 4.28 ± 0.05 |
| 2θ = 22.02 ± 0.2 | 4.04 ± 0.05 |
| 2θ = 22.77 ± 0.2 | 3.91 ± 0.05 |
| 2θ = 25.11 ± 0.2 | 3.55 ± 0.05 |
| 2θ = 25.52 ± 0.2 | 3.49 ± 0.05 |
| 2θ = 27.05 ± 0.2 | 3.30 ± 0.05 |
| 2θ = 30.27 ± 0.2 | 2.95 ± 0.05 |
| 2θ = 31.53 ± 0.2 | 2.48 ± 0.05 |

The IR spectrum of the crystalline Pyroxasulfone exhibited the functional group characteristic vibrations peaks at wavenumbers of one or more at about 2987.05, 1572.09, 1488.51, 1375.02, 1329.55, 1251.06, 1168.65, 1125.87, 1102.30 and 1050.31 $cm^{-1}$, as shown in Figure 2.

The Differential scanning calorimetry (DSC) of the crystalline Pyroxasulfone exhibited an endothermic melting peak with onset at 131.7°C and peak maximum at 133.3°C, with a melting enthalpy of 39.95 J/g, as shown in Figure 3.

**Example 3**

**Preparation of crystalline modification I of Pyroxasulfone (crystallization from n-butyl acetate)**

Pyroxasulfone prepared in Example 1 (10 g) was placed in a 3 neck round bottom flask, together with n-butyl acetate (60 mL) and stirred. The resulting slurry was heated to 90°C to achieve a homogeneous solution. The insoluble particles, if any, were removed by filtration. The remaining solution was slowly cooled to room temperature. Upon cooling, fine crystals formed. The slurry of crystals and solution was stirred at room temperature for 2 hours. Thereafter, the slurry was filtered and washed with n-butyl acetate (3 mL). The filtered crystals were dried under vacuum at room temperature in order to remove the n-butyl acetate traces from the crystalline product.

The crystalline product thus obtained had a purity of >98% and the product recovered as crystals was found to be not less than 80% yield.

The crystal product was analyzed by IR spectrometry, XRD and DSC and found to be the crystalline modification I of Pyroxasulfone.

**Example 4: Preparation of suspension concentrate (SC)**

Samples were prepared by mixing all the active ingredients and components listed in Table 2 uniformly and grinding with a Dyno-Mill (manufactured by Willy A. Bachofen AG) to obtain a suspension concentrate.

Table 2

| Sample | Active compound | Weight % of Pyroxasulfone | Components |
|---|---|---|---|
| S1 | Amorphous Pyroxasulfone (prepared in Example 1) | 40 | 13.00% Alkylnaphthalene sulfonic acid formaldehyde condensate (Morwet® D425); 15.00% Ethyleneoxide / propyleneoxide block copolymer (Pluronic® PE10500); 10.00% Alkylpolyvinylpyrrolidone, 5.00% Butylated hydroxytoluene (BHT), 3.00% Propylene glycol, 0.01% 1,2-Benzisothiazol-3(2H)-one (Proxel®); and balance to 100% with water |
| S2 | Pyroxasulfone, crystalline modification I from Example 2) | 40 | Same as above |
| S3 | Pyroxasulfone, crystalline modification I from Example3) | 40 | Same as above |

**Example 5: Preparation of Water Dispersible Granules (WG)**

Water dispersible granules (WG) were prepared as follows:
All the components listed in Table 3 below were mixed, blended and milled in a highspeed rotary mill. Sufficient water was added to obtain an extrudable paste. The paste was extruded through a die or screen to form an extrudate. The wet extrudate was dried at 70°C in a vacuum oven and then sifted through 0.71 mm to 2 mm screens to obtain the product granules.

Table 3

| Sample | Active compound | Weight % of Pyroxasulfone | Components |
|---|---|---|---|
| S4 | Amorphous Pyroxasulfone (prepared in Example 1) | 85 | 2.00% Alkyl naphthalene sulphonate, sodium salt (Akzo Nobel); 2.00% Lignosulfonic acid, sodium salt, REAX® 88B); 2.00% Naphthalenesulfonic acid, polymer with formaldehyde, sodium salt (TAMOL® NN8906), 2.00% Sucrose, 2.00% Non-ionic aqueous emulsion of Polydimethylsiloxanes, Mannitol balance to 100% |
| S5 | Pyroxasulfone, crystalline modification I from Example 2) | 85 | Same as above |
| S6 | Pyroxasulfone, crystalline modification I from Example3) | 85 | Same as above |

**Example 6: Dispersibility test**

0.3077 g of calcium carbonate and 0.092 g of magnesium oxide were dissolved in a small amount of diluted hydrochloric acid, then heated and boiled on a sand bath to remove hydrochloric acid. Distilled water was added to the remaining solution to make 10 liters in total. Subsequently, 100 ml of the obtained solution was put into a 100 ml stoppered measuring cylinder, and kept at 20° C in a temperature-controlled room. 100 mg samples of S4 to S6 were put into the measuring cylinder, and left to stand for 30 seconds. The measuring cylinder was then turned upside down repeatedly at a rate of once a second, whereupon the turning-down frequency (number of turns) needed for disintegrating the whole water-dispersible material was counted. The results are shown in Table 4.

Table 4

| Sample | turning-down frequency needed for disintegrating |
|---|---|
| S4 | 55 |
| S5 | 4 |
| S6 | 4 |

As can be seen from the results set out in Table 4 above, the crystalline modification I of Pyroxasulfone exhibits a significantly higher dispersibility than amorphous Pyroxasulfone.

**Example 7: Storage stability test**

Samples (S1, S2 and S3) were stored at 54 °C for 1 month, 3 months and 6 months. The procedures were followed according to CIPAC MT 46.3. The concentration of Pyroxasulfone was measured at the end of each storage time by HPLC. The aggregation was measured by observation. The original concentration of Pyroxasulfone in each formulation was 40 %. The results are listed in Table 5.

Table 5.

| Sample | 1 month | | 3 month | | 6 month | |
|---|---|---|---|---|---|---|
| | Concentration of Pyroxasulfone (%) | Aggregation | Concentration of Pyroxasulfone (%) | Aggregation | Concentration of Pyroxasulfone (%) | Aggregation |
| S1 | 30 | + | 25 | +++ | 20 | +++++ |
| S2 | 39 | - | 39 | - | 38 | - |
| S3 | 39 | - | 38 | - | 38 | - |
| Remark: "+" means small amount of observed aggregation; "+++++" means a significant amount of observed aggregation; "-" means no observed aggregation. | | | | | | |

As can be seen from the results set out in Table 5 above, the crystalline modification I of Pyroxasulfone exhibits a significantly higher storage stability than amorphous Pyroxasulfone. The crystalline modification I of Pyroxasulfone has also been demonstrated to exhibit a significantly lower tendency to aggregate during storage.

## Claims

**1.** A crystalline modification I of Pyroxasulfone, the crystalline modification exhibiting at least three of the following reflexes, in any combination, as 2θ±0.2 degree in an X-ray powder diffractogram (XRD) recorded using Cu-Kα radiation at 25 °C:

$$2\theta = 9.94 \pm 0.2 \quad (1)$$

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.02 \pm 0.2 \quad (5)$$

$$2\theta = 22.77 \pm 0.2 \quad (6)$$

$$2\theta = 25.11 \pm 0.2 \quad (7)$$

$$2\theta = 25.52 \pm 0.2 \quad (8)$$

$$2\theta = 27.05 \pm 0.2 \quad (9)$$

$$2\theta = 30.27 \pm 0.2 \quad (10)$$

$$2\theta = 31.53 \pm 0.2 \quad (11)$$

**2.** The crystalline modification I of Pyroxasulfone according to claim 1, wherein the crystalline modification exhibits at least three, more preferably four, still more preferably five, more preferably still six, especially seven, of the following reflexes, in any combination, as $2\theta \pm 0.2$ degree in an X-ray powder diffractogram (XRD) recorded using Cu-K$\alpha$ radiation at 25°C:

$$2\theta = 9.94 \pm 0.2 \quad (1)$$

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.02 \pm 0.2 \quad (5)$$

$$2\theta = 22.77 \pm 0.2 \quad (6)$$

$$2\theta = 25.11 \pm 0.2 \quad (7)$$

$$2\theta = 27.05 \pm 0.2 \quad (9)$$

$$2\theta = 30.27 \pm 0.2 \quad (10)$$

preferably wherein the crystalline modification exhibits all of the following reflexes, in any combination, as $2\theta \pm 0.2$ degree in an X-ray powder diffractogram (XRD) recorded using Cu-K$\alpha$ radiation at 25°C:

$$2\theta = 19.95 \pm 0.2 \quad (2)$$

$$2\theta = 20.36 \pm 0.2 \quad (3)$$

$$2\theta = 20.76 \pm 0.2 \quad (4)$$

$$2\theta = 22.77 \pm 0.2 \quad (6)$$

$$2\theta = 30.27 \pm 0.2 \quad (10)$$

**3.** A crystalline modification I of Pyroxasulfone, the crystalline modification exhibiting an infrared (IR) spectrum with characteristic functional group vibration peaks at wavenumbers ($cm^{-1}$, ± 0.2%) of one or more of 2987.05, 1572.09, 1488.51, 1375.02, 1329.55, 1251.06, 1168.65, 1125.87, 1102.30 and 1050.31 $cm^{-1}$.

**4.** A crystalline modification I of Pyroxasulfone, the crystalline modification exhibiting a melting point of from 131.7 to 134.1°C.

**5.** A crystalline modification I of Pyroxasulfone, the crystalline modification exhibiting a differential scanning calorimetry (DSC) profile having an endothermic melting peak with onset at 131.7°C and peak maximum at 133.3°C.

**6.** A composition for controlling plant growth, the composition comprising the crystalline modification I of Pyroxasulfone according to any preceding claim; preferably wherein the composition is in the form of a suspension concentrate (SC), an oil-based suspension concentrate (OD), water-soluble granules (SG), a dispersible concentrate (DC), an emulsifiable concentrate (EC), an emulsion seed dressing, a suspension seed dressing, granules (GR), microgranules (MG), a suspoemulsion (SE) or water-dispersible granules (WG).

**7.** Use of the crystalline modification I of Pyroxasulfone according to any of claims 1 to 5 in the control of undesirable plant growth.

**8.** A method for controlling plant growth at a locus, the method comprising applying to the locus the crystalline modification I of Pyroxasulfone according to any of claims 1 to 5 or a composition according to claim 6.

**9.** The use of claim 7 or the method of claim 8, wherein the plant growth being controlled is in a crop of plants selected from maize, soybeans, wheat, triticale, corn, cotton, beans, peanuts, potatoes, rape, garlic, tobacco, sunflower, castor-oil plants and scallion, preferably maize, soybeans, wheat and triticale; and/or wherein the undesired plants are selected from broadleaf plants and grassy weeds, preferably annual gramineous weeds, more preferably crab grass, green bristlegrass, goosegrass herb, barnyard grass, moleplant seed, alopecurus, wild oat, blue grass, stiff grass, and teff; and broadleaf weeds, more preferably chenopodiaceae, amaranthaceae, polygonaceae, daygrass, hamamelis, and dodder.

**10.** A method for preparing a crystalline modification I of Pyroxasulfone, the method comprising the steps of:

i) providing a solution of Pyroxasulfone in a solvent system comprising a one or more solvents, preferably wherein the Pyroxasulfone is amorphous Pyroxasulfone;
ii) precipitating the crystalline modification I of Pyroxasulfone from the solution; and
iii) isolating the precipitated crystalline modification I of Pyroxasulfone.

**11.** The method according to claim 10, wherein the solvent system comprises one or more solvents selected from ethers, preferably ethyl propyl ether, n-butyl ether, anisole, phenetole, cyclohexyl methyl ether, dimethyl ether, dimethyl glycol, diphenyl ether, dipropyl ether, diisopropyl ether, di-n-butyl ether, diisobutyl ether, diisoamyl ether, ethylene glycol dimethyl ether, isopropyl ethyl ether, methyl tert-butyl ether, dichlorodiethyl ether, polyethers of ethylene oxide and/or propylene oxide; esters, preferably malonates, n-butyl ester (n-butyl acetate), methyl acetate, isobutyl acetate, dimethyl carbonate, diethyl carbonate, dibutyl carbonate and ethylene carbonate; more preferably wherein the solvent system comprises one or more solvents selected from dichlorodiethyl ether, methyl tert-butyl ether, methyltetrahydrofuran, malonates, n-butyl acetate, isobutyl acetate, diethyl carbonate.

**12.** The method according to either of claims 10 or 11, wherein in step ii), the solution is cooled to a temperature of from -20 to 10°C.

**13.** The method according to any of claims 10 to 12, wherein in step ii) a vacuum is applied to the solution.

**14.** The method according to any of claims 10 to 13, wherein in step ii) seed crystals are added to the solution; preferably wherein the seed crystals are crystals of Pyroxasulfone, more preferably the crystalline modification I of Pyroxasulfone.

**15.** Use of a solvent system to prepare crystalline Pyroxasulfone having an improved stability and resistance to hydrolysis, wherein the solvent system comprises one or more solvents selected from ethers and esters; preferably wherein the solvent system comprises one or more solvents selected from dichlorodiethyl ether and n-butyl acetate.

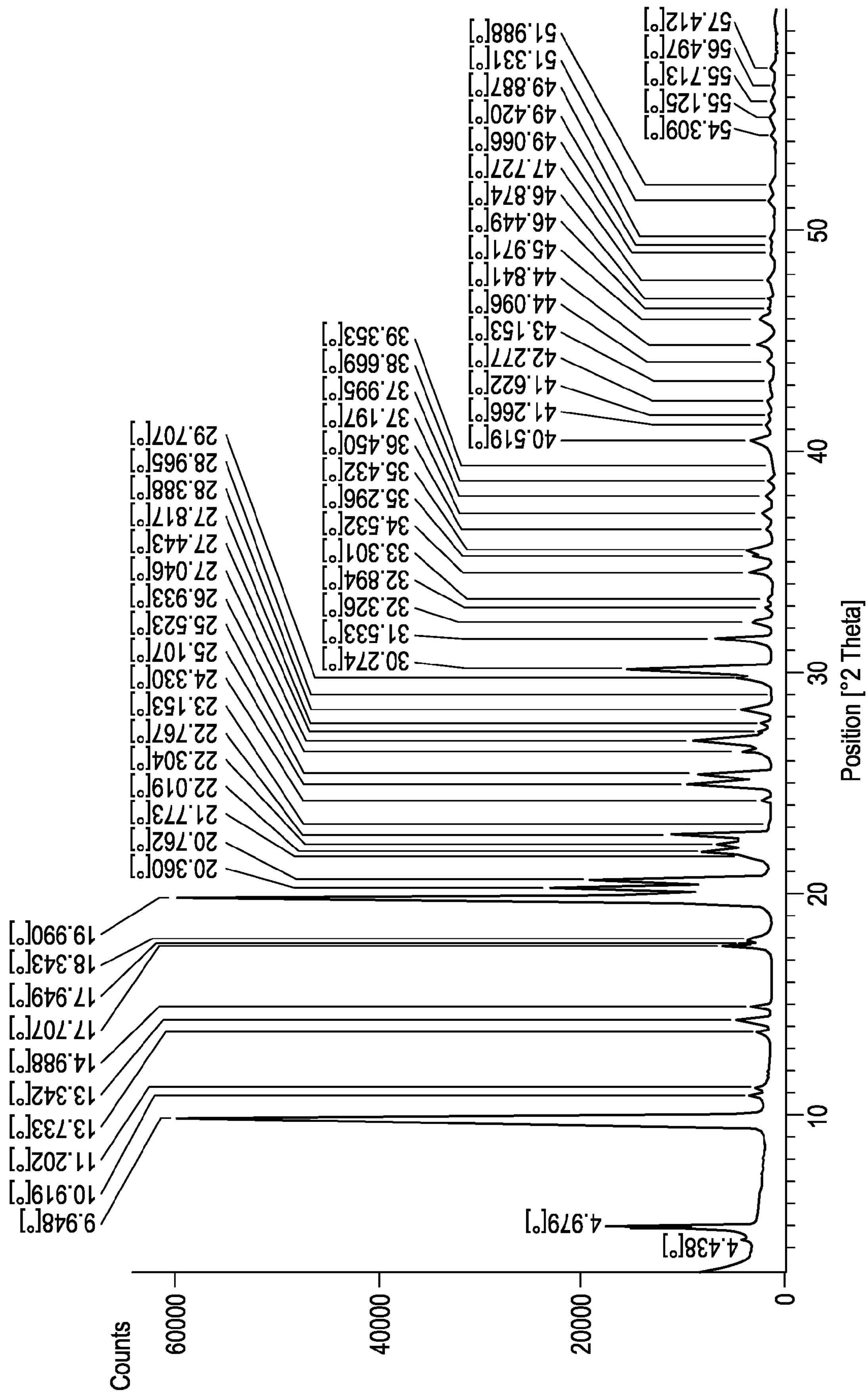
Counts
60000
40000
20000
0
Position [°2 Theta]
10
20
30
40
50
4.438[°]
4.979[°]
9.948[°]
10.919[°]
11.202[°]
13.733[°]
13.342[°]
14.988[°]
17.707[°]
17.949[°]
18.343[°]
19.990[°]
20.360[°]
20.762[°]
21.773[°]
22.019[°]
22.304[°]
22.767[°]
23.153[°]
24.330[°]
25.107[°]
25.523[°]
26.933[°]
27.046[°]
27.443[°]
27.817[°]
28.388[°]
28.965[°]
29.707[°]
30.274[°]
31.533[°]
32.326[°]
32.894[°]
33.301[°]
34.532[°]
35.296[°]
35.432[°]
36.450[°]
37.197[°]
37.995[°]
38.669[°]
39.353[°]
40.519[°]
41.266[°]
41.622[°]
42.277[°]
43.153[°]
44.096[°]
44.841[°]
45.971[°]
46.449[°]
46.874[°]
47.727[°]
49.066[°]
49.420[°]
49.887[°]
51.331[°]
51.988[°]
54.309[°]
55.125[°]
55.713[°]
56.497[°]
57.412[°]

Figure 1

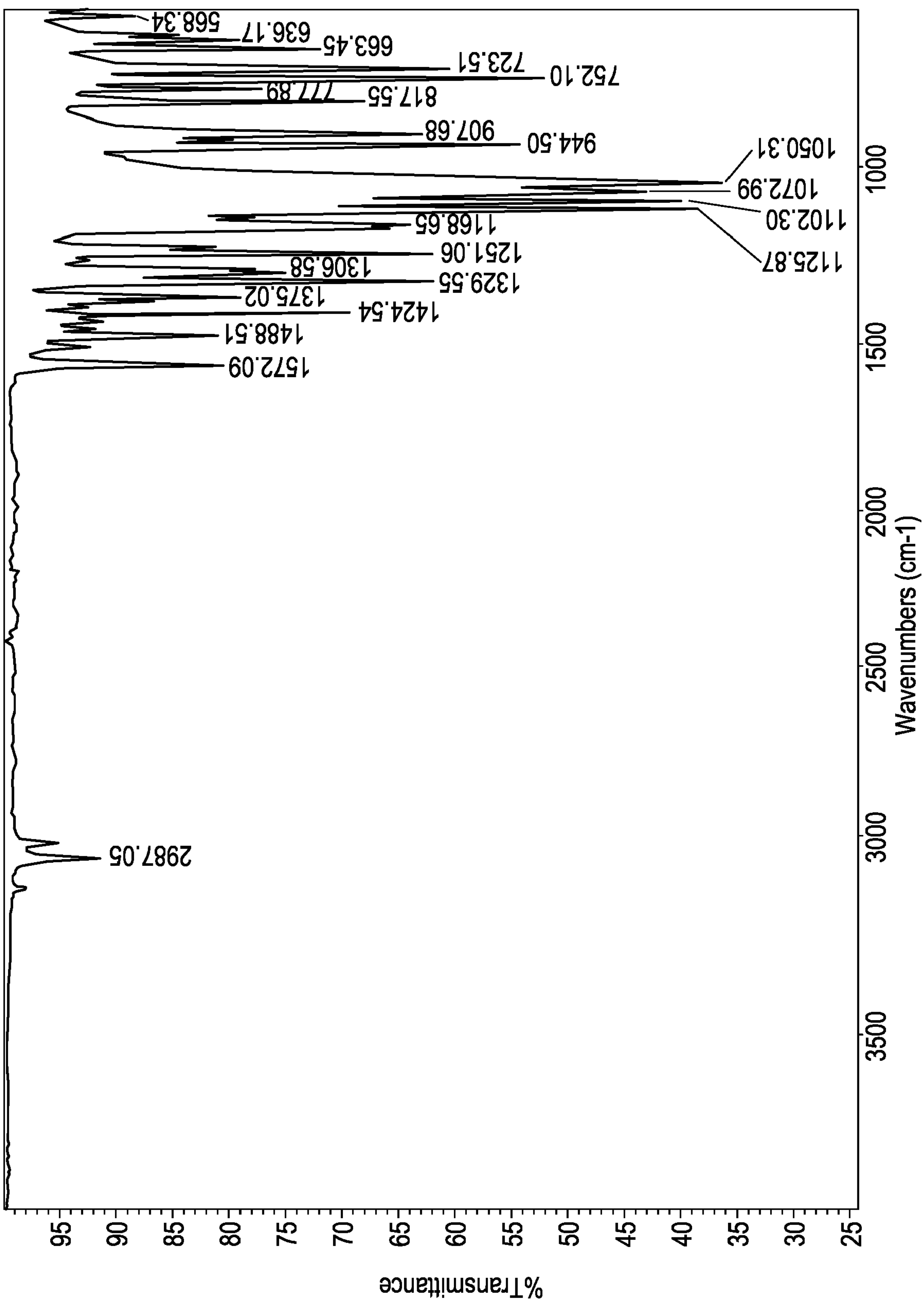
%Transmittance
95
90
85
80
75
70
65
60
55
50
45
40
35
30
25
Wavenumbers (cm-1)
3500
3000
2500
2000
1500
1000
2987.05
1572.09
1488.51
1424.54
1375.02
1329.55
1306.58
1251.06
1168.65
1125.87
1102.30
1072.99
1050.31
944.50
907.68
817.55
777.89
752.10
723.51
663.45
636.17
568.34

Figure 2

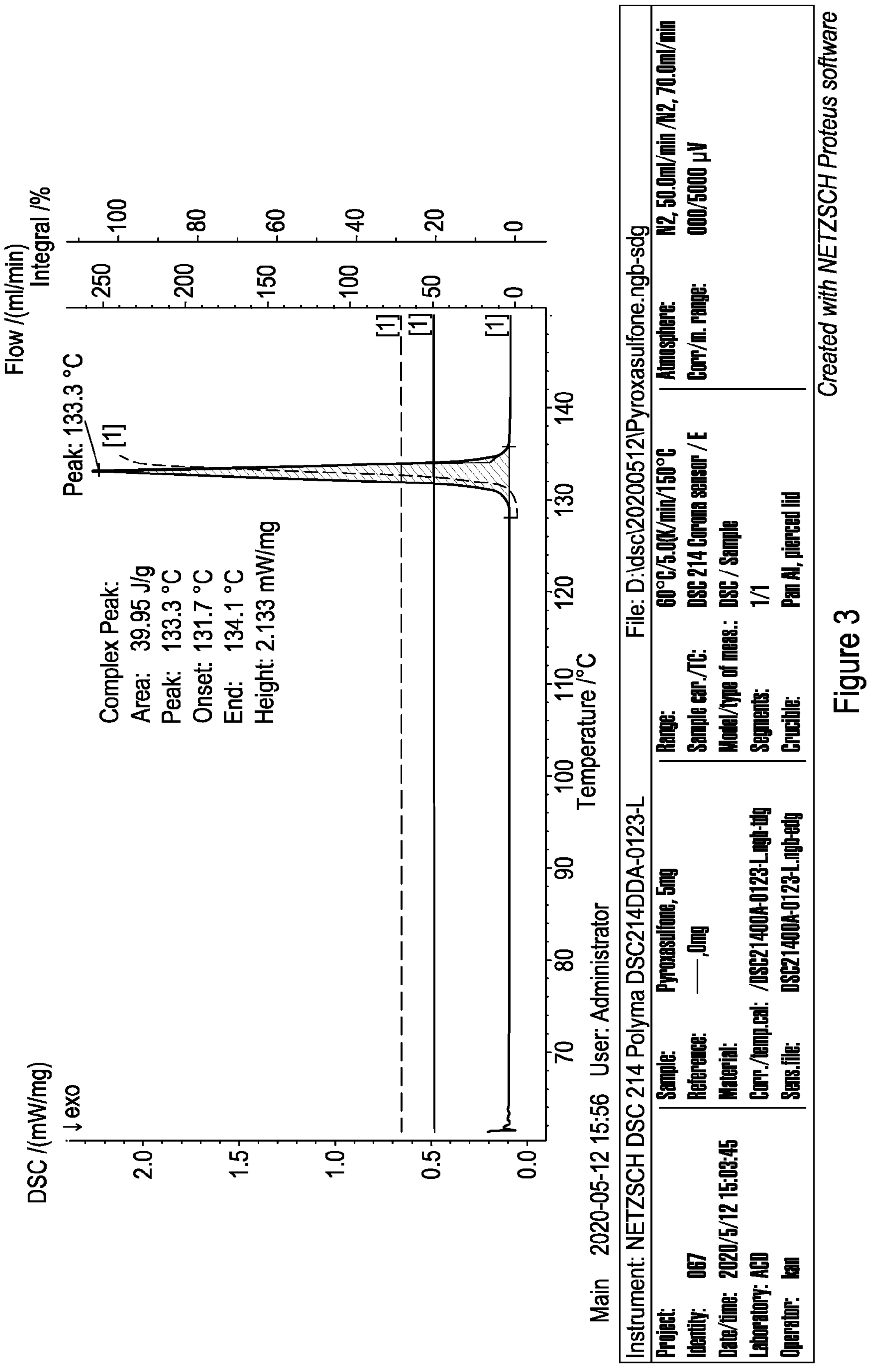

DSC /(mW/mg)
↓exo
2.0
1.5
1.0
0.5
0.0
Flow /(ml/min)
Integral /%
250
200
150
100
50
0
100
80
60
40
20
0
Complex Peak:
Area: 39.95 J/g
Peak: 133.3 °C
Onset: 131.7 °C
End: 134.1 °C
Height: 2.133 mW/mg
Peak: 133.3 °C
[1]
70
80
90
100
110
120
130
140
Temperature /°C
Main 2020-05-12 15:56 User: Administrator
Instrument: NETZSCH DSC 214 Polyma DSC214DDA-0123-L
File: D:\dsc\20200512\Pyroxasulfone.ngb-sdg
Project:
Identity: 067
Date/time: 2020/5/12 15:03:45
Laboratory: ACD
Operator: lan
Sample: Pyroxasulfone, 5mg
Reference: ——,0mg
Material:
Corr./temp.cal: /DSC21400A-0123-L.ngb-tdg
Sens.file: DSC21400A-0123-L.ngb-edg
Range: 60°C/5.0(K/min)/150°C
Sample car./TC: DSC 214 Corona sensor / E
Model/type of meas.: DSC / Sample
Segments: 1/1
Crucible: Pan Al, pierced lid
Atmosphere: N2, 50.0ml/min /N2, 70.0ml/min
Corr/m. range: 000/5000 µV
Created with NETZSCH Proteus software


Figure 3

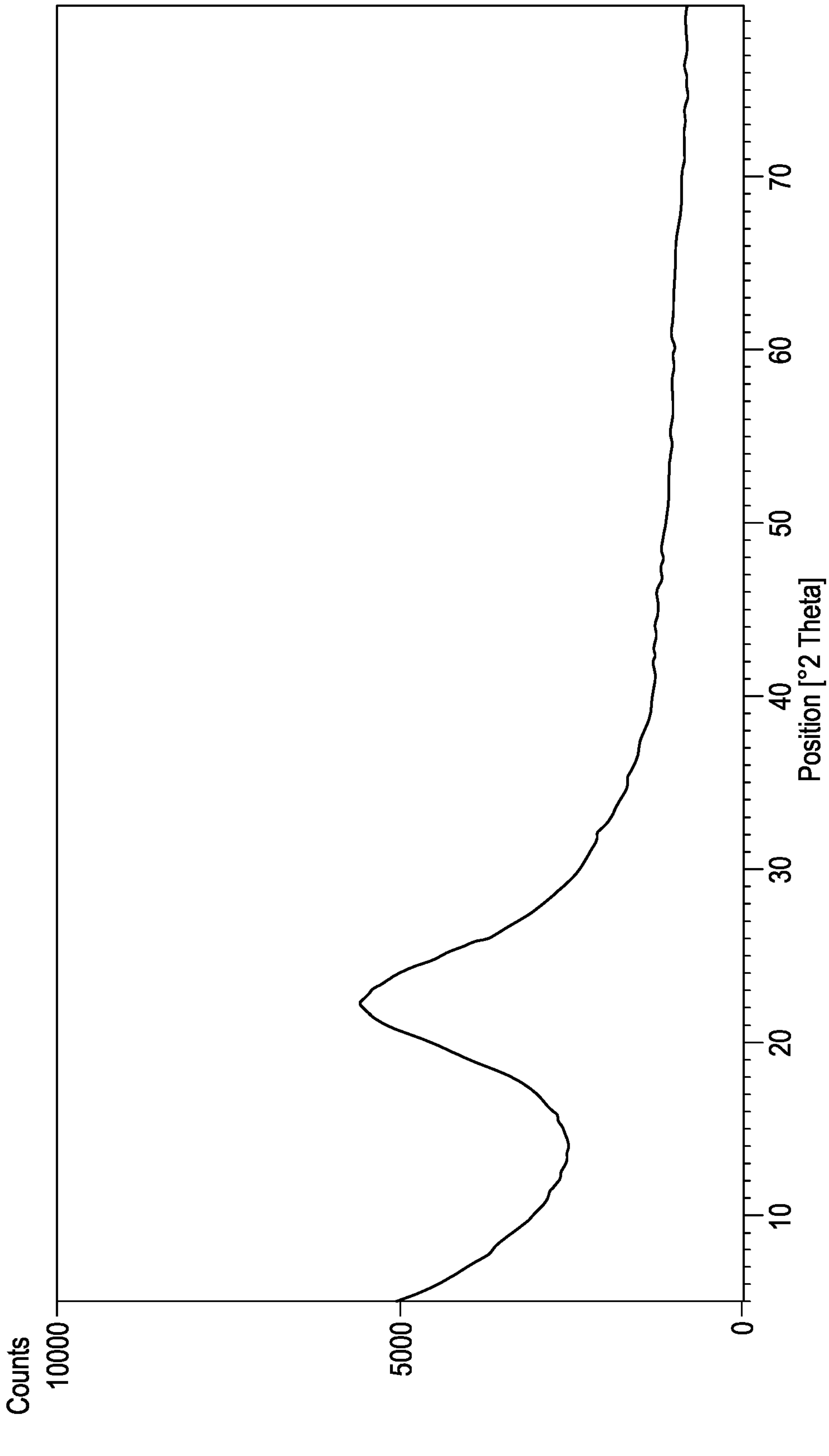


Figure 4

Europäisches Patentamt
European Patent Office
Office européen des brevets

# EUROPEAN SEARCH REPORT

Application Number
EP 22 15 0924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2021/002484 A2 (KUMIAI CHEMICAL INDUSTRY CO [JP]) 7 January 2021 (2021-01-07) * paragraphs [0479]-[0496], [0810], [0848], [0853] and [0859]; table on pages 261-262; claim 32 * | 1-15 | INV. C07D413/12 A01N43/80 |
| X | US 2005/256004 A1 (TAKAHASHI SATORU [JP] ET AL) 17 November 2005 (2005-11-17) * page 26; example 46 * | 15 | |
| A,D | US 7 256 298 B2 (KUMIAI CHEMICAL INDUSTRY CO [JP]) 14 August 2007 (2007-08-14) * column 33 - column 34; examples 1, 3 * | 1-15 | |
| X,P | WO 2021/144796 A1 (ADAMA AGAN LTD [IL]) 22 July 2021 (2021-07-22) * claims 1, 3, 6, 9, 10 * | 1-15 | |

TECHNICAL FIELDS SEARCHED (IPC)

C07D
A01N

The present search report has been drawn up for all claims

1

| Place of search | Date of completion of the search | Examiner |
|---|---|---|
| Munich | 4 April 2022 | Moriggi, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 15 0924

This annex lists the patent family members relating to the patent documents cited in the above-mentioned European search report. The members are as contained in the European Patent Office EDP file on
The European Patent Office is in no way liable for these particulars which are merely given for the purpose of information.

04-04-2022

| Patent document cited in search report | Publication date | Patent family member(s) | Publication date |
|---|---|---|---|
| WO 2021002484 A2 | 07-01-2021 | CN 112969697 A | 15-06-2021 |
| | | JP 6920572 B2 | 18-08-2021 |
| | | JP 2021178843 A | 18-11-2021 |
| | | JP WO2021002484 A1 | 13-09-2021 |
| | | TW 202132290 A | 01-09-2021 |
| | | WO 2021002484 A2 | 07-01-2021 |
| US 2005256004 A1 | 17-11-2005 | AR 040809 A1 | 20-04-2005 |
| | | AU 2003254863 A1 | 25-02-2004 |
| | | BR 0313241 A | 09-08-2005 |
| | | JP 4789101 B2 | 12-10-2011 |
| | | JP WO2004014138 A1 | 24-11-2005 |
| | | MY 132698 A | 31-10-2007 |
| | | PA 8579501 A1 | 04-02-2005 |
| | | PE 20040373 A1 | 27-08-2004 |
| | | PL 207304 B1 | 30-11-2010 |
| | | TW 200406154 A | 01-05-2004 |
| | | UA 78071 C2 | 15-02-2007 |
| | | US 2005256004 A1 | 17-11-2005 |
| | | UY 27909 A1 | 27-02-2004 |
| | | WO 2004014138 A1 | 19-02-2004 |
| US 7256298 B2 | 14-08-2007 | AU 2003252447 A1 | 23-02-2004 |
| | | BR 0313178 A | 14-06-2005 |
| | | CA 2494130 A1 | 12-02-2004 |
| | | CN 1678588 A | 05-10-2005 |
| | | CN 1995021 A | 11-07-2007 |
| | | EP 1541561 A1 | 15-06-2005 |
| | | ES 2519442 T3 | 07-11-2014 |
| | | IL 166341 A | 30-12-2010 |
| | | JP 4551764 B2 | 29-09-2010 |
| | | JP WO2004013106 A1 | 21-09-2006 |
| | | KR 20050026082 A | 14-03-2005 |
| | | MX 255058 B | 04-03-2008 |
| | | PL 218431 B1 | 31-12-2014 |
| | | RS 20050081 A | 21-09-2007 |
| | | RU 2315758 C2 | 27-01-2008 |
| | | TW I337997 B | 01-03-2011 |
| | | UA 79483 C2 | 25-06-2007 |
| | | US 2005215797 A1 | 29-09-2005 |
| | | US 2007249844 A1 | 25-10-2007 |
| | | WO 2004013106 A1 | 12-02-2004 |
| WO 2021144796 A1 | 22-07-2021 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2021002484 A
- US 7256298 B
- CN 111393427 A
- WO 2020240392 A